# EUROPEAN PATENT SPECIFICATION

(11) **EP 3 665 185 B1**
(45) Date of publication and mention of the grant of the patent: **04.10.2023**
(21) Application number: 18762149.5
(22) Date of filing: 03.08.2018
(51) Int. Cl.: C07K 14/005

(54) **RECOMBINANT PROTEIN VP60 AND PROCEDURE FOR ITS PRODUCTION AND PURIFICATION**
REKOMBINANTES PROTEIN VP60 UND VERFAHREN FÜR SEINE PRODUKTION UND REINIGUNG
PROTÉINE RECOMBINANTE VP60 ET PROCÉDURE POUR SA PRODUCTION ET PURIFICATION

(30) Priority: 07.08.2017 IT 201700091210
(43) Date of publication of application: 17.06.2020
(73) Proprietor: Istituto Zooprofilattico Sperimentale Dell'Umbria E Delle Marche, 06126 Perugia (PG) (IT)
(72) Inventor: DE GIUSEPPE, Antonio, 06126 Perugia (PG) (IT)
(74) Representative: Brunacci, Marco
(86) International application number: PCT/IB2018/055859
(87) International publication number: WO 2019/030633

(56) References cited:
- EP-A2- 0 704 529
- S LAURENT ET AL: "Structural, antigenic and immunogenic relationships between European brown hare syndrome virus and rabbit haemorrhagic disease virus.", JOURNAL OF GENERAL VIROLOGY., vol. 78, no. 11, 1 November 1997 (1997-11-01), pages 2803-2811, XP055461216, GB ISSN: 0022-1317, DOI: 10.1099/0022-1317-78-11-2803
- DONG-KUN YANG ET AL: "Rabbit Hemorrhagic Disease Virus Variant Recombinant VP60 Protein Induces Protective Immunogenicity", JOURNAL OF MICROBIOLOGY AND BIOTECHNOLOGY., vol. 25, no. 11, 28 November 2015 (2015-11-28), pages 1960-1965, XP055462009, KR ISSN: 1017-7825, DOI: 10.4014/jmb.1504.04002
- G. LE GALL ET AL: "European brown hare syndrome virus: molecular cloning and sequencing of the genome", JOURNAL OF GENERAL VIROLOGY., vol. 77, no. 8, 1 August 1996 (1996-08-01) , pages 1693-1697, XP055461548, GB ISSN: 0022-1317, DOI: 10.1099/0022-1317-77-8-1693 & DATABASE EMBL [Online] 1996, Database accession no. Z69620
- GUO HUIMIN ET AL: "Self-assembly of virus-like particles of rabbit hemorrhagic disease virus capsid protein expressed inEscherichia coliand their immunogenicity in rabbits", ANTIVIRAL RESEARCH, vol. 131, 23 April 2016 (2016-04-23), pages 85-91, XP029570505, ISSN: 0166-3542, DOI: 10.1016/J.ANTIVIRAL.2016.04.011
- CHIARI MARIO ET AL: "Red foxes (Vulpes vulpes) feeding brown hares (Lepus europaeus) infected by European brown hare syndrome virus (EBHSv) might be involved in the spread of the virus", EUROPEAN JOURNAL OF WILDLIFE RESEARCH, SPRINGER, DE, vol. 62, no. 6, 8 October 2016 (2016-10-08), pages 761-765, XP036104307, ISSN: 1612-4642, DOI: 10.1007/S10344-016-1055-4 [retrieved on 2016-10-08] -& DATABASE EMBL [Online] 13 October 2016 (2016-10-13), "European brown hare syndrome virus isolate Bs15_2 capsid protein VP60 gene, partial cds.", XP002779439, retrieved from EBI accession no. EM_STD:KU961678 Database accession no. KU961678
- XUEXING ZHENG ET AL: "Development of a VLP-based vaccine in silkworm pupae against rabbit hemorrhagic disease virus", INTERNATIONAL IMMUNOPHARMACOLOGY, vol. 40, 1 November 2016 (2016-11-01), pages 164-169, XP055461655, NL ISSN: 1567-5769, DOI: 10.1016/j.intimp.2016.08.016

## Description

### Technical Field

The present invention relates to a procedure for the production and purification of a recombinant protein VP60.

### Background Art

To date, the need is particularly felt to manage the spread of Brown Hare haemorrhagic syndrome (EBHS) from a health and environmental point of view.

This syndrome is caused by a Calicivirus, belonging to the family Caliciviridae, genus Lagovirus.

The Calcivirus viral genome comprises a single strand of RNA having a length between 7.5 kb and 8 kb.

As is known, Calciviruses have a capsid consisting of a single capsidic protein, the so-called VP60 (viral protein 60) having a molecular weight of about 60 kDa and a diameter of between 35 and 40 nm.

The capsidic protein VP60 of EBHS self-assembles spontaneously to form so-called "Virus-Like Particles" (VLPs) which, as is known, are particles without genetic material but morphologically and antigenically structured in the same way as mature virions.

The spread of EBHS syndrome occurs via the oral-nasal route, either through direct contact between infected animals or through indirect contact, i.e. mediated by transmission vectors such as insects, or contaminated instruments in common use by humans, such as cages.

Due to the high environmental resistance of the virus, the latter can be transmitted in the faeces of predator animals feeding on infected hares, or alternatively in the faeces of carcasses of the latter which have died of EBHS. Numerous studies report that morbidity can reach 100% in a seronegative population, and mortality usually reaches 30-50% in at least three-months-old animals.

It is easy to appreciate how the strong spread of EBHS syndrome together with high infectivity and mortality make it one of the major health problems facing hare breeding.

Vaccine prophylaxis is the only effective control method for EBHS syndrome, however, the absence of commercially available vaccines prevents its implementation.

To at least partially overcome these problems, procedures have been developed for expression in a heterologous (baculovirus) system of the recombinant protein VP60 capable of self-assembling to form VLPs having immunogenic power with respect to EBHS syndrome.

In particular, the VPLs produced to date have long and laborious purification procedures.

To this must be added the fact that the recombinant protein, once extracted and isolated, needs to be purified.

Furthermore, the VLPs formed by aggregation of the recombinant proteins have complex production processes which provide, in fact, a plurality of operating phases comprising purification and/or concentration phases that need to be performed in series with each other.

To this must be added the fact that the purification phases of the VLPs are extremely complex and affect the final outcome of the purification itself.

### Description of the Invention

The main aim of the present invention is to provide a procedure for the production and purification of a recombinant protein VP60 that greatly facilitates the production process of same, simplifying its purification.

Described herein but not part of the present invention is to provide a recombinant protein VP60. The present invention is a procedure for the production and purification which ensure adequate self-assembly to give stable VLPs.

The related procedure for the production and purification allows overcoming the aforementioned drawbacks of the prior art within the scope of a simple, rational, easy, efficient to use and cost-effective solution.

The aforementioned objects are achieved by the present procedure for the production and purification having the characteristics of claims 1 to 8.

### Brief Description of the Drawings

Other characteristics and advantages of the present invention will become more evident from the description of recombinant protein VP60, which is not part of the invention, and related procedure for the production and purification, illustrated by way of an indicative, but non-limiting example, in the attached drawings in which:

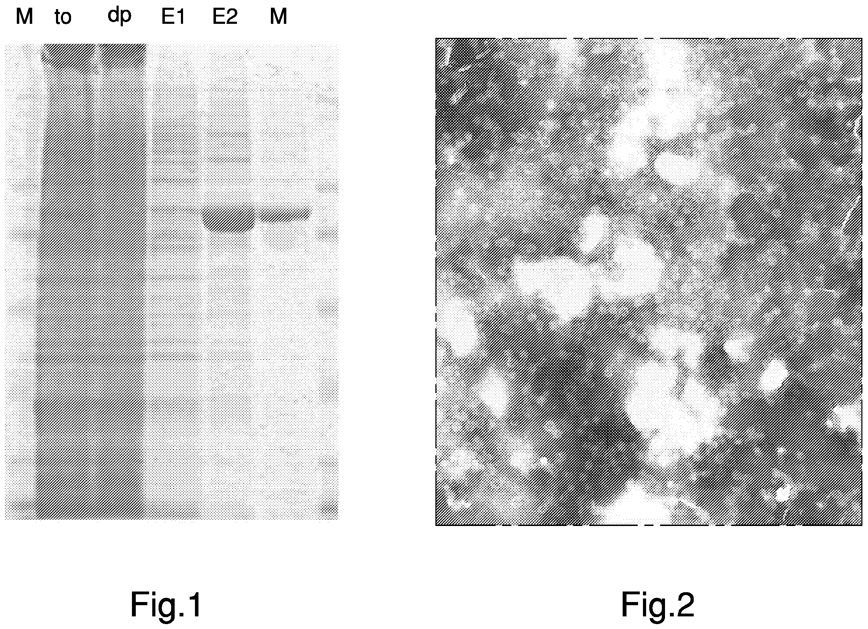

Figure 1 is a representation of the SDS-PAGE analysis performed on the E-VP60-His₆ fractions purified by immobilized metal affinity chromatography or IMAC.

More specifically, the SDS-PAGE analysis relates to the recombinant protein E-VP60-His₆ purified by affinity chromatography with His-Select Nickel Affinity Gel resin. In detail, (t0) corresponds to a cellular extract; (dp) corresponds to a protein extract after incubation with chromatographic resins; (E1) corresponds to the first elution fraction; (E2) corresponds to the second elution fraction, (E3) corresponds to the third elution fraction and (M) corresponds to the marker. Figure 2 is a photographic specimen obtained from the electronic microscope and showing self-assembled VLPs from E-VP60-His₆.

### Embodiments of the Invention

Described herein but not part of the invention is a recombinant protein VP60 comprising an amino acid sequence presenting a polyhistidine tail at the carboxy-terminal end.

It is an anti-EBHS recombinant protein VP60. Preferably, the polyhistidine tail is composed of six histidine residues.

It cannot however be ruled out that the polyhistidine tail consists of seven, eight, nine, etc. histidine residues.

Prefereably, embodiment, the recombinant protein VP60 has the following amino acid sequence (SEQ ID NO:1).

This means that the anti-EBHS recombinant protein VP-60 has the following amino acid sequence (SEQ. ID NO:1)

MEGKPRADAPGTATTASVPGTTTDGMDPGVVASTDVVTADNVAASV
ATAGIGGPPQQVSPQESWRANFFYNDVFTWSVTDAPGSILYTVQHSPQ
NNPFTQVLSQMYAGWAGGMQFRFIVAGSGIFGGRLVCAIIPPGIQIQPG
LEVRQFPHVVIDARSLEPVTITMPDLRPEMYHPTGNPGLVPTLVVSVYN
NLINPFGGTTSAIQVTVETRPSEDFEFVLIRAPSSKTVDSVNPSWLLTTP
VLTGAGSDNRWGAPIVGLQPVPGGFSTSNRHWNMNGETYGWSSPRFD
DIDHPSGNVSYPTGSATNTIETWYANAGTATTNPISNVAPDGFPDMGAI
PFSGSTIPTGAWVGFGQVWNASNGTPYVGTVQAYELGFASGAPSNVR
PVTTTTGAQLVAKSIYGVAIAQNQSSAGIIFMSKGMVSTPGVAATTYTP
QPSAIVTTPGTPVAAPIGKNTPIMFSAVVRRTGDVNAGPGSANGTQYG
VGSQPLSVTLGLSLTNYSSALQPGQFFVWQLNFASGFMEIGLNTDGYF
YAGTGAYSGMIELTDLIDVRPVGVRPNTSTLVFNLTGVATTGYSYVAG
HHHHHH
SEQ ID NO:1

The present invention relates to a procedure for the production and purification of the recombinant protein.

Such procedure comprises an isolation phase of a nucleotide sequence encoding for the protein VP60 from a viral strain of EBHS.

The viral strain is isolated from an outbreak of infection that occurred in a limited environment, in this case the Umbria-Marche territory.

The procedure then continues with an extraction phase of viral RNA and reverse transcription of the viral RNA to obtain a sequence of encoding cDNA.

The RNA extraction phase is performed starting from a sample according to what is known from the QIAamp^{®} Viral RNA Mini Handbook (QIAGEN) kit execution protocol.

According to a preferred embodiment of the procedure, the sample from which to extract the RNA is equal to about 5-10 mg of tissue, in this case 200 µL of liver, lung and spleen homogenate.

Once an RNA sample has been obtained, the extraction phase comprises an elution phase of the extracted RNA sample.

According to a preferred embodiment, the obtained RNA sample is eluted with 14 µL of elution buffer.

From the 14 µL, 5 µL are taken and then denatured at a working temperature of substantially 65°C for an operating time of substantially 5 minutes.

This is followed by the retro-transcription phase which involves the use of start sequences (primers) chosen by the group comprising:
5'-GATAGTCTCGAGGCCACCATGGAGGGTAAGCCTCGGGCTG-3' (SEQ ID NO: 3), 5'-CCTAGGCCGGCGACATAGGAATATCCAGTGGT-3' (SEQ ID NO: 4), 5'-GAGCCCGACAATTGGTGCACC-3' (SEQ ID NO: 5), 5' -CAGACAACAGGTGGGGTGCAC-3' (SEQ ID NO:6).

Preferably, according to a preferred embodiment of the invention, the start nucleotide sequences comprise the above specific nucleotide sequences respectively for the restriction sites of ApaLI and NgoMIV: GTGCAC and GCCGGC respectively.

More specifically, the complete start sequences for the respective NgoMiv-R, ApaLI-R, ApaLI-F restriction sites are shown below.

**Table 1**

| Primer | Sequence 5' → 3' |
|---|---|
| **EBHS-VP60-F** | GATAGTCTCGAGGCCACCATGGAGGGTAAGC CTCGGGCTG |
| **EBHS-VP60/NgoMiv-R** | CCTAGGCCGGCGACATAGGAATATCCAGTGG T |
| **EBHS-VP60/ApaLI-R** | GAGCCCGACAATTGGTGCACC |
| **EBHS-VP60/ApaLI-F** | CAGACAACAGGTGGGGTGCAC |

Preferably, the retro-transcription phase takes place by means of a reverse transcriptase enzyme of the SuperScript ^{™} II Reverse Transcriptase of Invitrogen (Life technologies) type.

Afterwards, once a single-stranded cDNA sequence has been obtained, the procedure comprises an amplification phase of the sequence itself.

The amplification phase is performed by setting up a polymerase chain reaction, the so-called PCR, which, as the expert in the sector knows, involves the use of a DNA polymerase.

With reference to a preferred embodiment, the DNA polymerase used is of the AccuPrime^{™} *Pfx* DNA Polymerase (Life technologies) type.

In detail, the amplification phase comprises a plurality of operating phases which, in turn, comprise a first denaturation operating phase at a working temperature of substantially 95°C and for an operating time of substantially 2 minutes.

This is followed by the amplification phase which comprises a second denaturation operating phase at a working temperature of substantially 95°C and for an operating time of substantially 15 seconds.

At this point, the amplification phase comprises a third operating adhesion phase of the start sequences to the cDNA filament at a working temperature of substantially 58°C for an operating time of substantially 30 seconds.

The start sequences (primers) used in the adhesion phase are selected from the group shown in Table 1.

Preferably, the start sequences (primers) used are EBHS-VP60-F in combination with EBHS-VP/ApaLI-R, and EBHS-VP/ApaLI-F in combination with VP60/NgoMiv-R.

Furthermore, the amplification phase comprises a fourth extension operating phase at a working temperature of substantially 68°C for an operating time of substantially 1 minute.

More specifically, the second denaturation operating phase, the third adhesion operating phase and the fourth extension operating phase are repeated 35 times in sequence.

Finally, the procedure comprises a final extension phase at a working temperature of substantially 68°C and for an operating time of substantially 5 minutes to obtain a plurality of amplified nucleotide sequences.

At this point, the procedure comprises a first ligation phase of the nucleotide sequences amplified in a pCR Blunt vector and the subsequent transformation of the competent cells of *E. Coli* with the obtained constructs.

Subsequently, the procedure comprises a restriction and nucleotide sequence analysis phase of the constructs bearing the amplified nucleotide sequences related to the 5' and 3' ends, respectively of the gene encoding for the protein VP60 of the EBHS.

Once the two inserts have been obtained, they are bound together to obtain a nucleotide sequence, i.e. the complete gene, encoding for the protein VP60 and having the polyhistidine amino acid tail at the carboxy-terminal end of the protein.

It is pointed out that in the present treatise the term "gene" relates to a nucleotide sequence encoding for a protein.

It may, but does not necessarily, comprise elements found in and/or associated with a gene encoding for that protein in nature.

In order to obtain the expression of the recombinant protein, hereinafter indicated by the expression "E-VP60-His₆", the procedure comprises a cloning phase of the nucleotide sequence provided with the polyhistidine tail and encoding for E-VP60-His₆ in a baculovirus transfer vector.

Preferably, the aforementioned baculovirus transfer vector is of the pFastBac1 and/or pOET2C-6xHis type.

In detail, the procedure comprises a first cloning phase of the nucleotide sequence provided with the polyhistidine tail and encoding for E-VP60-His₆ in pFastBac1.

Afterwards, the procedure comprises a cleavage phase mediated by restriction enzymes such as EcoRI and HindIII and finally a second cloning phase in pOET2C-6xHis to obtain a plasmid identified hereunder as E-VP60-His/pOET2C.

Below is the sequence analysis carried out on E-VP60-His₆.

ATGGAGGGTAAGCCTCGGGCTGACGCCCCTGGTACCGCTACCACAG
CATCTGTTCCTGGGACCACTACCGACGGCATGGACCCAGGGGTGGTG
GCCAGCACTGACGTTGTGACGGCTGACAACGTTGCAGCATCTGTTGC
CACTGCGGGGATTGGAGGACCACCACAACAGGTCTCACCTCAAGAG

TCTTGGCGTGCGAACTTCTTTTACAATGATGTATTTACCTGGTCAGTC
ACCGATGCACCCGGTAGCATTCTTTACACAGTTCAACACTCCCCGCA
AAACAACCCCTTCACGCAGGTGCTGAGCCAAATGTATGCTGGTTGGG
CTGGTGGCATGCAGTTTAGATTCATAGTTGCTGGGTCTGGTATTTTTG
GCGGAAGGCTCGTGTGTGCAATCATACCACCTGGGATCCAGATACA
ACCTGGGCTTGAGGTTAGACAGTTTCCTCATGTTGTCATCGATGCAC
GCTCACTTGAGCCAGTGACTATAACAATGCCTGATCTTCGTCCTGAA
ATGTACCACCCAACAGGCAATCCTGGACTTGTCCCAACACTCGTCGT
GAGTGTGTACAACAACTTGATCAACCCCTTTGGGGGTACCACCAGTG
CAATTCAGGTTACTGTTGAGACTAGGCCAAGTGAGGACTTTGAGTTT
GTGTTGATCCGCGCACCTTCAAGTAAAACAGTGGACTCAGTGAACCC
CTCGTGGCTCTTGACAACTCCGGTCTTGACCGGTGCTGGTTCAGACA
ACAGATGGGGTGCACCAATTGTCGGGCTCCAGCCCGTACCAGGTGG
CTTCTCCACTTCCAACAGGCACTGGAACATGAATGGTGAAACCTACG
GTTGGTCCAGCCCCAGGTTTGACGACATTGATCATCCAAGTGGCAAT
GTATCCTACCCCACTGGTAGTGCAACCAACACCATTGAGACCTGGTA
TGCCAATGCTGGAACAGCTACCACCAACCCAATCTCCAACGTAGCAC
CAGACGGGTTTCCTGACATGGGAGCCATCCCGTTCAGTGGCTCAACC
ATCCCGACTGGTGCCTGGGTTGGGTTTGGGCAGGTGTGGAACGCCTC
CAATGGCACCCCATACGTGGGGACCGTTCAGGCGTACGAGCTGGGG
TTCGCAAGCGGGGCCCCGTCCAACGTTCGTCCTGTCACAACAACCAC
GGGTGCGCAGCTGGTTGCAAAGTCGATCTATGGGGTTGCCATTGCCC
AAAACCAATCCAGTGCTGGCATTATCTTCATGTCGAAAGGGATGGTC
TCAACACCTGGGGTGGCTGCCACAACATACACCCCGCAACCAAGCG
CAATTGTCACAACACCTGGAACACCTGTGGCTGCACCCATTGGCAAG
AATACACCTATTATGTTCAGTGCAGTCGTGCGGAGGACTGGTGATGT
GAACGCCGGTCCTGGGTCCGCTAACGGCACACAGTATGGCGTTGGCT
CGCAGCCATTGTCAGTAACACTTGGACTGTCCCTGACAAACTACTCC
TCCGCACTTCAACCTGGCCAGTTCTTTGTGTGGCAACTCAACTTCGCT
TCTGGCTTCATGGAGATTGGTTTGAATACAGATGGTTACTTTTATGCA
GGGACTGGTGCTTACTCAGGCATGATTGAATTGACAGACCTCATTGA

TGTGCGGCCCGTGGGAGTCCGGCCAAACACGAGCACACTCGTGTTCA
ACCTTACCGGAGTGGCCACCACTGGATATTCCTATGTCGCCGGTCAT
CATCACCATCACCATTGA
SEQ ID NO:2

Also described herein but not part of the invention is a baculovirus plasmid vector comprising the nucleic acid molecule (SEQ ID NO:2) encoding for E-VP60-His₆.

More specifically, the above plasmid vector comprises a nucleotide sequence containing specific sequences of Baculovirus optimized for transformation into E. Coli.

More specifically, and as mentioned above, the plasmid vector is a baculovirus transfer vector.

Advantageously, the above nucleic acid molecule consists of the nucleotide sequence SEQ ID NO:2.

Furthermore, the procedure comprises an expression and synthesis phase of the protein E-VP60-His₆.

In detail, the plasmid E-VP60-His/pOET2C was used for the generation of the recombinant baculovirus expressing the recombinant protein E-VP60-His₆.

The expression and synthesis phase comprises a transfection phase of Sf21 insect cells grown in monolayer on a HYQ SFX-Insect serum-free medium (Hyclone).

The Sf21 insect cells were transfected with linearized flashBAC^{™} DNA and plasmid DNA E-VP60-His/pOET2C according to what is known from the Oxford Expression Technologies' execution protocol.

In the case in question, the Sf21 insect cells were transfected with 100 ng of linearized flashBAC^{™} DNA and 500 ng of plasmid DNA E-VP60-His/pOET2C.

After a 5-day incubation period at 27°C, the cell supernatant containing the recombinant baculovirus (P1 viral stock) was collected and used for the production of a small-scale high titre viral stock (P2 viral stock).

P2 viral stock was subsequently used for the production of a large-scale high titre viral stock (P3 viral stock).

At this point, the expression and synthesis phase comprises a production phase of the recombinant protein E-VP60-His₆.

The production phase comprises an infection phase of Sf21 insect cells with the previously obtained recombinant baculovirus, i.e. obtained from the transfection phase.

The production phase takes place by means of the culture of Sf21 insect cells inside rotating bottles in serum-free ExCell medium (SIGMA) with the addition of 100 µg/ml of streptomycin, 0.25 µg/ml of amphotericin B and 10 µg/ml of gentamycin.

In detail, the Sf21 insect cells are sown inside a number of rotating bottles at a concentration of 1.1 × 10⁶ cells/ml (200 × 10⁶ cells in 180 ml of ExCell Medium per rotating bottle) and infected with 0.1-1 recombinant baculovirus plaque forming units (PFUs) per cell.

Subsequently, the production phase comprises an incubation phase for an operating time of substantially three days.

At the end of the three days, the infected cells contained in the medium and the cells grown in adhesion to the wall of the rotating bottles are collected.

For each rotating bottle used, the medium containing the cells in suspension is then divided into 4×50 ml falcon tubes, and centrifuged at about 300 x g at a working temperature of substantially +4°C, and for an operating time of substantially 10 minutes.

Instead, the cells attached to the wall of the rotating bottles are washed with 10 ml of PBS.

Afterwards, the supernatant is eliminated and the cell pellets are washed with the 10 ml of PBS recovered from the washing of the cells attached onto the rotating bottles.

At this point, the cells are centrifuged again at about 300 x g at a working temperature of substantially 4°C for an operating time of substantially 7 minutes. After which the PBS is removed and the cells undergo lysis.

In this regard, it is specified that the present invention also relates to a heterologous system of expression of the prokaryotic or eukaryotic type. Preferably, the heterologous expression system of the prokaryotic type comprises vectors selected from the group including *E. Coli and Bacillus subtilitis.*

Alternatively, advantageously, the heterologous expression system of the eukaryotic type comprises vectors selected from the group including: yeasts such as e.g. *S. cerevisiae and Pichia pastoris,* or culture cells such as e.g. insect and mammal cells, or else plant models.

According to a preferred embodiment, the expression vector is of the recombinant Baculovirus type.

Finally, once the expression of the recombinant protein has been obtained, the procedure comprises a phase of extraction and purification of same.

The extraction and purification phase of the protein E-VP60-His₆ comprises a lysis phase wherein the infected Sf21 cells attached onto the walls of the rotating bottles are suspended in a specific lysis buffer, in this case equal to 10 ml.

In detail, the lysis buffer comprises 50 mM NaH₂PO₄/Na₂HPO₄ at pH8, 300 mM NaCl, 1% Triton X-100.

Afterwards, to the cell pellets contained in the 4 falcon tubes, a further lysis buffer is added for a total of another 10 ml.

At this point, the two cell lysate fractions are combined in a single tube for a total of 20 ml of lysate.

The fact is underlined that the cell lysates obtained from the union of the two fractions can be stored at a temperature of substantially -20°C until the next phase of the procedure.

In this respect, after the lysis phase, the procedure comprises a phase of extraction of the proteins E-VP60-His₆.

The cell lysate undergoes centrifugation at 16000 x g at a working temperature of substantially 4°C and for an operating time of substantially 30 minutes.

The next step is the recovery of the supernatant and the purification phase.

In detail, the purification phase is carried out by means of an affinity chromatography.

Preferably, the lysate is purified by means of only one affinity chromatography. Advantageously, the affinity chromatography is of the IMAC type.

In other words, the recombinant protein E-VP60-His₆ to which the present invention relates is purified by a single passage through the chromatographic column described below.

In fact, the preparation of the above chromatography column provides for the use of an agarose granule matrix (6%) charged with Nickel ions specifically binding the histidines of the proteins (HIS-Select^{®} HF Nickel Affinity Gel, SIGMA ALDRICH).

Purification is performed according to what is known from the HIS-Select^{®} HF Nickel Affinity Gel (SIGMA ALDRICH) execution protocol under native conditions.

Such execution protocol is, furthermore, implemented by means of the following solutions:
- the final bed of the column of 0.25 ml (per number of cells deriving from a rotating bottle), this indicates the amount of resin used for each rotating bottle; and
- addition of imidazole to the cell lysate in a final concentration of 10 mM.

Figure 1 shows the unexpected results relating to the qualitative and quantitative aspects of the recombinant protein E-VP60-His₆ purified by means of the above affinity chromatography.

Figure 1 shows all the protein fractions, starting from the cell lysate up to the elution fractions of the purified antigen. In particular, the obtained protein fractions underwent electrophoresis on acrylamide gels under denaturing conditions (SDS-PAGE), and were subsequently dyed with Coomassie-Blu.

More specifically: the line (t0) represents the cell lysate of the Sf21 infected with the recombinant baculovirus expressing the E-VP60-His₆; the line (dp) corresponds to the previous cell lysate after passing over the chromatographic resin; the lines (E1), (E2) and (E3) represent the first, second and third elution fractions of the purified recombinant protein E-VP60-His₆, respectively; finally, the line (M) indicates the protein marker for determining the molecular weight of the purified molecule.

The speed and ease of application of the procedure to which the present invention relates, ensures the production and purification of the E-VP60-His₆ also in a scale-up plant aimed at the large-scale production of the recombinant protein E-VP60-His₆ itself.

To this must be added that, besides the quantitative aspect inherent in the high yield of the product obtained starting from the cell lysate, the high degree of purity of the E-VP60-His₆ is evident.

At this point, the extraction and purification phase comprises a dialysis phase at a pH value between 4.5 and 5.5 with a solution comprising 140 mM NaCl, 2.68 mM KCl, 1.7 mM KH₂PO₄, 1.8 mM Na₂HPO₄, 9 mM NaH₂PO₄ (H₂0).

In particular, the fact is underlined that the use of solutions having a pH higher than 6.0 hinders the formation of VLP.

By contrast, pH values between 4.5 and 5.5 promote the formation of stable VLPs.

Furthermore, it is noticed that the fact that E-VP60-His₆ self-assembles to form VLPs makes possible its use in pharmaceutical compositions for vaccine purposes.

Also described herein but not part of the invention is a pharmaceutical composition comprising Virus Like Particles (VLPs) obtained from the nucleic acid molecule described above, and more specifically from the self-assembly of the recombinant proteins E-VP60-His₆.

It has in practice been ascertained how the described invention achieves the intended objects.

The fact is underlined that the special solution of providing a polyhistidine tail at the carboxy-terminal end of the recombinant protein E-VP60-His₆ makes it possible to purify the latter by means of only one affinity chromatography, thus considerably increasing the final purification yield.

## Claims

1. Procedure for the production and purification of a recombinant protein VP60 causing Brown Hare haemorrhagic syndrome (EBHS) comprising an amino acid sequence presenting a polyhistidine tail at the carboxy-terminal end, the procedure comprises at least the following phases:
- isolation of a nucleotide sequence encoding for a protein VP60 from a viral strain of EBHS, in turn, comprising the steps of:
- extraction of viral RNA and of reverse transcription of said viral RNA to obtain a sequence of cDNA;
- amplification of said sequence of cDNA in a plurality of fragments;
- cloning of said amplified sequence of cDNA in a transfer vector encoding said polyhistidine tail;
- expression and synthesis of said recombinant protein; and
- extraction and purification of said recombinant protein;
**characterised by** the fact that said extraction and purification phase comprises an affinity chromatography wherein said extraction and purification phase comprises at least one dialysis phase at a pH value comprised between 4.5 and 5.5 with a solution comprising NaCl, KCl, KH2PO4, Na2HPO4, NaH2PO4 and HCl.

2. Procedure according to claim 1 , **characterized by** the fact that said polyhistidine tail consists of six histidine residues.

3. Procedure according to one or more of the preceding claims, **characterized by** the fact that said recombinant protein presents the amino acid sequence SEQ ID NO:1.

4. Procedure according to any of the preceding claims, **characterized by** the fact that recombinant protein is encoded from a transfer vector.

5. Procedure according to any of the preceding claims, **characterized by** the fact that said transfer vector comprises a nucleotide sequence consisting in SEQ ID NO:2.

6. Procedure according to any of the preceding claims, **characterized by** the fact that said nucleotide sequence is cloned in a transfer vector of the type of baculovirus transfer vectors pBastBac1 and/or pOET2C-6xHis.

7. Procedure according to claim 1, **characterised by** the fact that said extraction and purification phase comprises only one affinity chromatography.

8. Procedure according to claim 7, **characterised by** the fact that said affinity chromatography is of the IMAC type.

9. Procedure according to one or more of the preceding claims, **characterised by** the fact that said amplification phase is mediated by at least one starting sequence selected from the list comprising: 5'-GATAGTCTCGAGGCCACCATGGAGGGTAAGCCTCGGGCTG-3', 5'-CCTAGGCCGGCGACATAGGAATATCCAGTGGT-3', 5'-GAGCCCGACAATTGGTGCACC-3', 5'-CAGACAACAGGTGGGGTGCAC-3'.

## Patentansprüche

1. Verfahren zur Herstellung und Reinigung eines rekombinanten Proteins VP60, das das Brown Hare haemorrhagic syndrome (EBHS) verursacht, umfassend eine Aminosäuresequenz, die einen Polyhistidinschwanz am carboxyterminalen Ende aufweist, wobei das Verfahren mindestens die folgenden Phasen umfasst:
- Isolierung einer Nukleotidsequenz, die für ein Protein VP60 kodiert, aus einem EBHS-Virusstamm, was wiederum die folgenden Schritte umfasst:
- Extraktion von viraler RNA und reverse Transkription der viralen RNA, um eine Sequenz von cDNA zu erhalten;
- Amplifikation der Sequenz der cDNA in eine Vielzahl von Fragmenten;
- Klonierung der amplifizierten cDNA-Sequenz in einem Transfervektor, der für den Polyhistidinschwanz kodiert,
- Expression und Synthese des rekombinanten Proteins; und
- Extraktion und Reinigung des rekombinanten Proteins;
**dadurch gekennzeichnet,**
**dass** die Extraktions- und Reinigungsphase eine Affinitätschromatographie umfasst, wobei die Extraktions- und Reinigungsphase mindestens eine Dialysephase bei einem pH-Wert zwischen 4,5 und 5,5 mit einer NaCl, KCl, KH2PO4, Na2HPO4, NaH2PO4 und HCl aufweisenden Lösung umfasst.

2. Verfahren nach Anspruch 1, **dadurch gekennzeichnet, dass** der Polyhistidinschwanz aus sechs Histidinresten besteht.

3. Verfahren nach einem oder mehreren der vorhergehenden Ansprüche, **dadurch gekennzeichnet, dass** das rekombinante Protein die Aminosäuresequenz SEQ ID NO:1 aufweist.

4. Verfahren nach einem der vorangehenden Ansprüche, **dadurch gekennzeichnet, dass** das rekombinante Protein von einem Transfervektor kodiert wird.

5. Verfahren nach einem der vorangehenden Ansprüche, **dadurch gekennzeichnet, dass** der Transfervektor eine Nukleotidsequenz, bestehend aus SEQ ID NO:2, umfasst.

6. Verfahren nach einem der vorhergehenden Ansprüche, **dadurch gekennzeichnet, dass** die Nukleotidsequenz in einen Transfervektor vom Typ der Baculovirus-Transfervektoren pBastBad und/oder pOET2C-6xHis kloniert wird.

7. Verfahren nach Anspruch 1, **dadurch gekennzeichnet, dass** die Extraktions- und Reinigungsphase nur eine Affinitätschromatographie umfasst.

8. Verfahren nach Anspruch 7, **gekennzeichnet durch** die Tatsache, dass die Affinitätschromatographie vom IMAC-Typ ist.

9. Verfahren nach einem oder mehreren der vorhergehenden Ansprüche, **dadurch gekennzeichnet, dass** die Amplifikationsphase durch mindestens eine Ausgangssequenz vermittelt wird, die aus der Liste ausgewählt ist, die umfasst:
5'- GATAGTCTCGAGGCCACCATGGAGGGTAAGCCTCGGGCTG-3',
5'- CCTAGGCCGGCGACATAGGAATATCCAGTGGT-3',
5'- GAGCCCGACAATTGGTGCACC-3',
5'- CAGACAACACAGGTGGGGTGCAC-3'.

## Revendications

1. - Procédé de production et de purification d'une protéine recombinante VP60 provoquant le syndrome hémorragique du lièvre brun (EBHS) comprenant une séquence d'acides aminés présentant une queue polyhistidine au niveau de l'extrémité carboxy-terminale, le procédé comprenant au moins les phases suivantes :
- isolement d'une séquence nucléotidique codant pour une protéine VP60 à partir d'une souche virale d'EBHS, comprenant à son tour les étapes de :
- extraction d'ARN viral et transcription inverse dudit ARN viral pour obtenir une séquence d'ADNc ;
- amplification de ladite séquence d'ADNc en une pluralité de fragments ;
- clonage de ladite séquence amplifiée d'ADNc dans un vecteur de transfert codant pour ladite queue polyhistidine ;
- expression et synthèse de ladite protéine recombinante ; et
- extraction et purification de ladite protéine recombinante ;
**caractérisé par le fait que** ladite phase d'extraction et de purification comprend une chromatographie d'affinité, ladite phase d'extraction et de purification comprenant au moins une phase de dialyse à une valeur de pH comprise entre 4,5 et 5,5 avec une solution comprenant NaCl, KCl, KH2PO4, Na2HPO4, NaH2PO4 et HCl.

2. - Procédé selon la revendication 1, **caractérisé par le fait que** ladite queue polyhistidine consiste en six résidus histidine.

3. - Procédé selon l'une ou plusieurs des revendications précédentes, **caractérisé par le fait que** ladite protéine recombinante présente la séquence d'acides aminés SEQ ID NO : 1.

4. - Procédé selon l'une quelconque des revendications précédentes, **caractérisé par le fait que** la protéine recombinante est codée à partir d'un vecteur de transfert.

5. - Procédé selon l'une quelconque des revendications précédentes, **caractérisé par le fait que** ledit vecteur de transfert comprend une séquence nucléotidique consistant en SEQ ID NO : 2.

6. - Procédé selon l'une quelconque des revendications précédentes, **caractérisé par le fait que** ladite séquence nucléotidique est clonée dans un vecteur de transfert du type des vecteurs de transfert baculovirus pBastBac1 et/ou pOET2C-6xHis.

7. - Procédé selon la revendication 1, **caractérisé par le fait que** ladite phase d'extraction et de purification comprend une seule chromatographie d'affinité.

8. - Procédé selon la revendication 7, **caractérisé par le fait que** ladite chromatographie d'affinité est du type IMAC.

9. - Procédé selon l'une ou plusieurs des revendications précédentes, **caractérisé par le fait que** ladite phase d'amplification est médiée par au moins une séquence de départ choisie dans la liste comprenant : 5'-GATAGTCTCGAGGCCACCATGGAGGGTAAGCCTCGGGCTG-3', 5'-CCTAGGCCGGCGACATAGGAATATCCAGTGGT-3', 5'-GAGCCCGACAATTGGTGCACC-3', 5'-CAGACAACAGGTGGGGTGCAC-3'.
